# EUROPÄISCHE PATENTSCHRIFT

(11) **EP 1 517 890 B1**
(45) Veröffentlichungstag und Bekanntmachung des Hinweises auf die Patenterteilung: **16.07.2008**
(21) Anmeldenummer: 03735534.4
(22) Anmeldetag: 04.06.2003
(51) Int. Cl.: C07D 205/08, C07D 487/08, A61K 31/397, A61P 3/06, A61P 9/10

(54) **KATIONISCH SUBSTITUIERTE DIPHENYLAZETIDINONE, VERFAHREN ZU DEREN HERSTELLUNG, DIESE VERBINDUNGEN ENTHALTENDE ARZNEIMITTEL UND DEREN VERWENDUNG**
CATIONICALLY SUBSTITUTED DIPHENYL AZETIDINONES, METHOD FOR THEIR PRODUCTION, MEDICAMENTS CONTAINING SAID COMPOUNDS AND USE THEREOF
DIPHENYLACETINIDONES A SUBSTITUTION CATIONIQUE, PROCEDE DE FABRICATION, MEDICAMENTS CONTENANT CES COMPOSES ET UTILISATION

(30) Priorität: 19.06.2002 DE 10227507
(43) Veröffentlichungstag der Anmeldung: 30.03.2005
(73) Patentinhaber: Sanofi-Aventis Deutschland GmbH, 65929 Frankfurt am Main (DE)
(72) Erfinder: JAEHNE, Gerhard, 65929 Frankfurt (DE); FRICK, Wendelin, 65510 Hünstetten-Beuerbach (DE); FLOHR, Stefanie, CH-4153 Reinach (CH); LINDENSCHMIDT, Andreas, 65812 Bad Soden (DE); GLOMBIK, Heiner, 65719 Hofheim (DE); KRAMER, Werner, 55130 Mainz-Laubenheim (DE); HEUER, Hubert, 55270 Schwabenheim (DE); SCHAEFER, Hans-Ludwig, 65239 Hochheim (DE)
(86) Internationale Anmeldenummer: PCT/EP2003/005814
(87) Internationale Veröffentlichungsnummer: WO 2004/000803

(56) Entgegenhaltungen:
- WO-A-02/18432
- WO-A-02/50027
- WO-A-02/50060
- WO-A-02/50068

## Beschreibung

Die Erfindung betrifft kationisch substituierte Diphenylazetidinone, deren physiologisch verträgliche Salze sowie physiologisch funktionelle Derivate.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685) und US 5,756,470].

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, sehr gering resorbierbar sind. Unter sehr gering resorbierbar wird eine intestinale Resorption kleiner 10%, bevorzugt kleiner oder gleich 5% verstanden.

Die neuen Verbindungen sollen insbesonders eine geringere Resorption als Ezetimibe auf weisen.
Bei geringerer Resorption zeigen pharmazeutische Wirkstoffe in der Regel deutlich weniger Nebenwirkungen.

Die Erfindung betrifft daher Verbindungen der Formel 1, 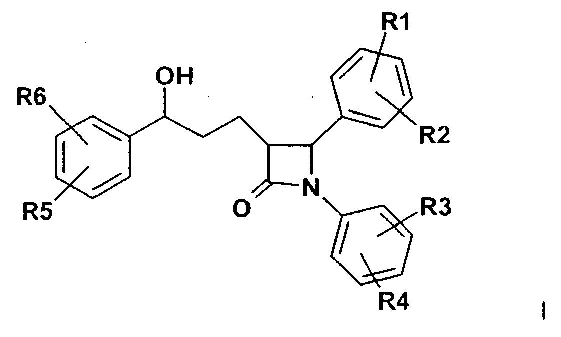 worin bedeuten
- R1, R2, R3, R4, R5, R6: unabhängig voneinander (C₀-C₃₀)-Alkylen-(LAG)ₙ, wobei n = 1 - 5 sein kann und wobei ein oder mehrere C-Atome des Alkylenrests durch -S(O)ₙ-, mit n = 0 - 2, -O-, -(C=O)-, -(C=S)-, -CH=CH-, -C≡C-, -N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂ SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- (LAG)ₙ: mono-, bi- oder tricyclischer Trialkylammonium-Rest, mono-, bi- oder tricyclischer Trialkylammoniumalkyl-Rest, -(CH₂)₀₋₁₀-C(=NH)(NH₂), - (CH₂)₀₋₁₀-C(=NH)(NHOH) oder -NR7-C(=NR8)(NR9R10) und R7, R8, R9 und R10 unabhängig voneinander H, (C₁-C₆)-Alkyl, (C₁-C₆)-Alkyl-Phenyl, Phenyl, (C₃-C₈)-Cycloalkyl) sein können und n = 1 - 5 sein kann;
wobei immer mindestens einer der Reste R1 bis R6 die Bedeutung (C₀-C₃₀)-Alkylen-(LAG)ₙ, wobei n = 1 - 5 ist und wobei ein oder mehrere C-Atome des Alkylenrests durch -S(O)ₙ-, mit n = 0 - 2, -O-, -(C=O)-, -(C=S)-, -CH=CH-, -C≡C-, - N((C₁-C₆)-Alkyl)-, -N(Phenyl)-, -N((C₁-C₆)-Alkyl-Phenyl)-, -N(CO-(CH₂)₁₋₁₀-COOH)-oder -NH- ersetzt sein können, besitzen muß,
sowie deren pharmazeutisch verträglichen Salze.

Bevorzugt sind Verbindungen der Formel I, worin mindestens einer der Reste R1 bis R6 die Bedeutung (C₀-C₃₀)-Alkylen-(LAG), wobei ein oder mehrere C-Atome des Alkylenrests durch -O-, -(C=O)-, -N((C₁-C₆)-Alkyl)- ,-N(CO-(CH₂)₁₋₁₀-COOH)- oder - NH- ersetzt sein können, besitzt.

Besonders bevorzugt sind Verbindungen der Formel I, worin einer der Reste R1 oder R3 die Bedeutung (C₀-C₃₀)-Alkylen-(LAG) hat, wobei ein oder mehrere C-Atome des Alkylenrests durch -O-, -(C=O)-, -N(CH₃)-, oder -NH- ersetzt sein können.

Ganz besonders bevorzugt sind Verbindungen der Formel I, worin einer der Reste R1 oder R3 die Bedeutung -(CH₂)₀₋₁-Y-W-(C₀-C₂₅)-Alkylen-Y'-W'-(LAG) hat; worin ein oder mehrere C-Atome des Alkylenrests durch O-Atome ersetzt sein können und wobei Y und W unabhängig voneinander NH, NCH₃, C=O, O, eine Bindung oder S(O)ₙ, mit n = 0 - 2, sein können und Y' und W' unabhängig voneinander NH, NCH₃, C=O, O, eine Bindung oder S(O)ₙ, mit n = 0 - 2, sein können oder Y-W oder Y'-W' jeweils für sich zusammen genommen eine Bindung sein kann.

Weiterhin bevorzugt sind Verbindungen der Formel I, worin die Gruppe LAG ein bicyclischer Trialkylammoniumalkyl-Rest ist.

Unter einem mono- oder bi- oder tricyclischen Trialkylammonium-Rest werden z.B. Reste wie 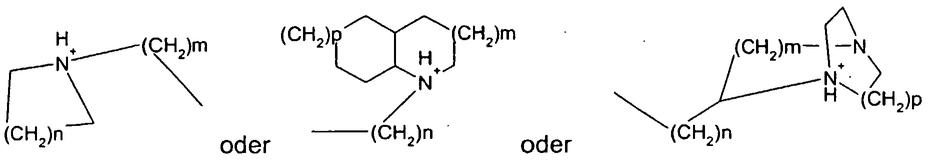 verstanden, wobei n, m und p unabhängig voneinander 0 - 10 sein kann und eine oder mehrere CH₂-Gruppen unabhängig voneinander durch O, S(O)ₙ, wobei n = 0 - 2 sein kann, NH, N-(C₁-C₁₀)-Alkyl, N-Phenyl oder N-CH₂-Phenyl ersetzt sein können.

Unter einem mono- oder bicyclischen Trialkylammoniumalkyl-Rest werden z.B. Reste wie 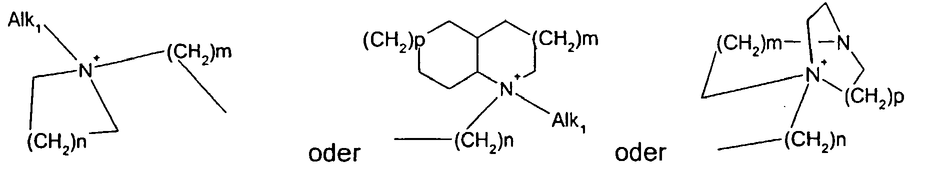 oder 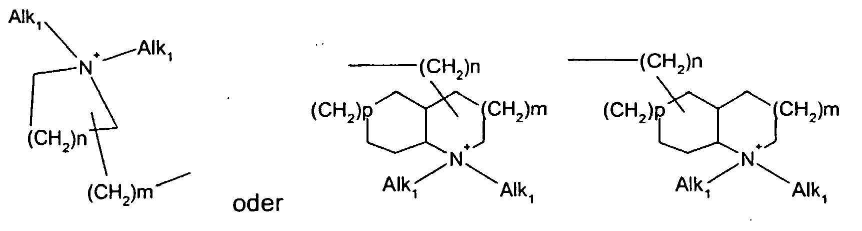 oder 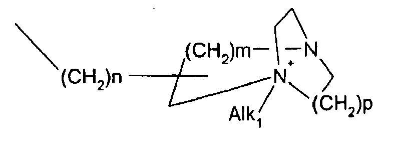 verstanden, wobei n, m und p unabhängig voneinander 0 - 10 sein kann und eine oder mehrere CH₂-Gruppen unabhängig voneinander durch O, S(O)ₙ, wobei n = 0 - 2 sein kann, NH, N-(C₁-C₁₀)-Alkyl, N-Phenyl oder N-CH₂-Phenyl ersetzt sein können und Alk₁einen geraden oder verzweigten Alkylrest mit 1 bis 20 Kohlenstoffatomen bedeutet.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-; Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoffen verabreicht werden.

Die Menge einer Verbindung gemäß Formel (1), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Diphenylazetidinon-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.
Antidiabetika umfassen Insulin und Insulinderivate, wie z.B. Lantus^{®} oder HMR 1964, GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffinrechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor , wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Gliclazid, verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinyl-methoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliazid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC-3- oder MC-4-Agonisten, Orexin-Agonisten, H3-Agonisten, TNF-Agonisten, CRF-Agonisten, CRF BP-Antagonisten, Urocortin-Agonisten, *β*3-Agonisten, MCH (Melanin-konzentrierendes Hormon) Antagonisten, , CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-*β-*Agonisten verabreicht.

Bei einer Ausführungsform der Erfindung ist der weitere Wirkstoff Leptin.
Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen, wie z.B. Caromax^{®} verabreicht. Die Kombination mit Caromax® kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden. Die Kombination von Verbindungen der Formel I mit Caromax® zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel 1, als auch die reinen Stereoisomere der Formel 1, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

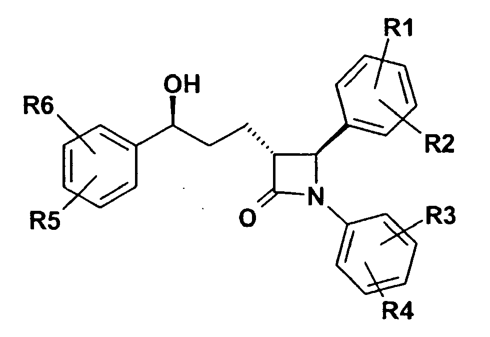

Als Aminoschutzgruppen werden bevorzugt der durch katalytische Hydrierung abspaltbare Benzyloxycarbonyl-(Z-)Rest, der durch schwache Säuren abspaltbare 2-(3,5-Dimethyloxyphenyl)propyl(2)oxycarbonyl (Ddz-) oder Trityl- (Trt)-Rest, der durch Säuren wie 3M Salzsäure abspaltbare t-Butylcarbamat (BOC-)-Rest und der durch sekundäre Amine abspaltbare 9-Fluorenylmethyloxycarbonyl- (Fmoc)-Rest herangezogen.

Die Erfindung betrifft weiterhin ein Verfahren zur Herstellung von Diphenylazetidinonderivaten der Formel I.

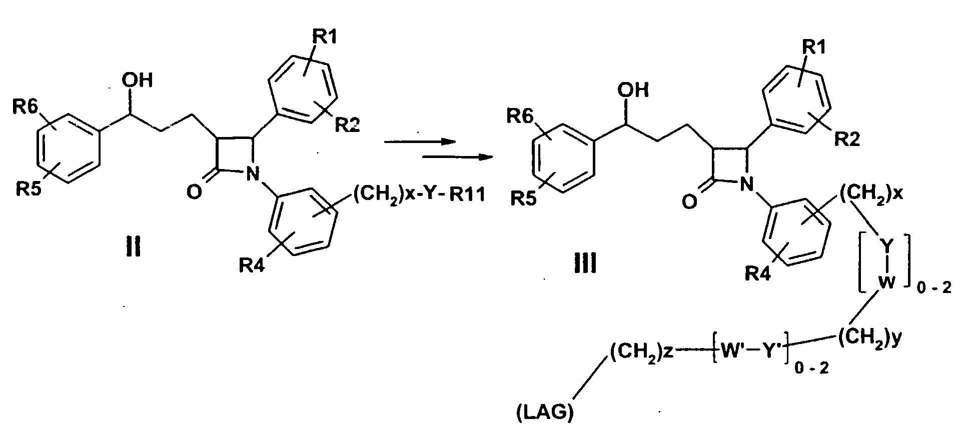

Y kann S, O, (C=O), (C=S), CH=CH, C≡C, N((C₁-C₆)-Alkyl), N(Phenyl), N((C₁-C₆)-Alkyl-Phenyl), N(CO-(CH₂)₁₋₁₀-COOH) oder NH bedeuten;
R11 kann H oder im Falle, dass Y = (C=O) oder (C=S) ist, OH bedeuten;
W, Y' und W' können, unabhängig voneinander und von Y, -S(O)ₙ-, mit n = 0 - 2, -O-, -(C=O)-, -(C=S)-, -CH=CH-, -C≡C-, -N((C₁-C₆)-Alkyl)-, -N(Phenyl), -N((C₁-C₆)-AlkylPhenyl)-, -N(CO-(CH₂)₁₋₁₀-COOH)- oder -NH- oder eine Bindung bedeuten;
x, y und z können unabhängig voneinander 0 bis 10 bedeuten.

Die Verknüpfung von -(CH₂)x-Y-R11 in Verbindung II kann alternativ auch an einem der anderen beiden Phenylringen sein.

Das Verfahren zur Herstellung der Verbindungen der Formel I ist dadurch gekennzeichnet, daß man z.B. ein Amin oder eine Hydroxy-Verbindung der Formel II mit einem Alkylierungs- oder einem Acylierungsreagenz umsetzt, das bevorzugt in omega-Position eine weitere Funktionalität - evtl. in geschützter Form - trägt. Diese wird (nach Entschützung) zur Anknüpfung der (LAG) verwendet, beispielsweise unter Ausbildung von Ether-, Amin oder Amidbindungen.

Die nachfolgenden Beispiele dienen zur näheren Erläuterung der Erfindung, ohne dieselbe auf in den Beispielen beschriebene Produkte und Ausführungsformen einzuschränken.

### Beispiel I

### 4-{1-(4-Fluor-phenyl)-3-[3-(4-fluor-phenyl)-3-hydroxy-propyl]-4-oxo-azetidin-2-yl}-N-hydroxy-benzamidin (3):

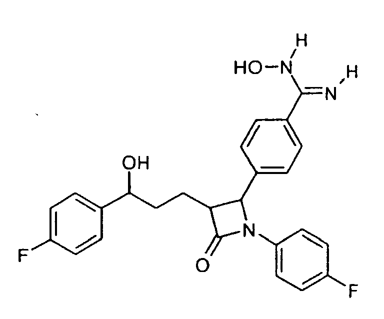

a) 4-[5-(4-Fluor-phenyl)-1-(4-fluor-phenylamino)-5-hydroxy-2-(2-oxo-4-phenyl-oxazolidin-3- carbonyl)-pentyl]-benzonitril (1):
   2.5 g 3-[5-(4-Fluor-phenyl)-5-hydroxy-pentanoyl]-4-phenyl-oxazolidin-2-on werden in 30 ml Dichlormethan unter Argon gelöst, dazu gibt man 3.9 g 4-[(4-Fluorphenylimino)-methyl]-benzonitril und kühlt auf -10°C.Zu dieser Mischung gibt man 6.4 ml Diisopropylethylamin und innerhalb von 30 min 4.05 ml Trimethylsilylchlorid, so dass die Temperatur -5°C nicht übersteigt. Bei dieser Temp. wird 1 Std. nachgerührt und dann auf -25°C gekühlt. Dann werden 0.8 ml Titantetrachlorid langsam zugegeben. Die dunkle Mischung wird über Nacht bei - 25 bis -30°C gerührt danach mit 35 ml 7proz. Weinsäurelösung zersetzt und 1 Std. bei Raumtemp. nachgerührt. Anschließend gibt man 15 ml einer 20%igen Natriumhydrogencarbonatlösung dazu und rührt erneut 1 Std. Nach Phasentrennung wird die org. Phase mit 30 ml Waser gewaschen, über Magnesiumsulfat getrocknet und auf ca. 10 ml eingeengt. Nach Zugabe von 2 ml Bistrimethylsilylacetamid erwärmt man 30 min. zum Rückfluss und engt danach i.Vak. ein. Der Rückstand wir d mit Ethylacetat/Heptan zur Kristallisation gebracht. Man saugt ab und trocknet i.Vak. Man erhält das Produkt mit dem Molekulargewicht 653.81 (C₃₇H₃₇F₂N₃O₄Si); MS (ESI⁺): 654.3 (M+H⁺), 582.2 (M+H⁺-Si(CH₃)₃).
b) {1-(4-Fluor-phenyl)-3-[3-(4-fluor-phenyl)-3-hydroxy-propyl]-4-oxo-azetidin-2-yl}-benzonitril (2):
   2 g 4-[5-(4-Fluor-phenyl)-1-(4-fluor-phenylamino)-5-hydroxy-2-(2-oxo-4-phenyl-oxazolidin-3- carbonyl)-pentyl]-benzonitril (1) werden in 20 ml Methyl-tert.-butyl-ether gelöst und mit 100 mg Tetrabutyl- ammoniumfluorid-Trihydrat und 1.3 ml Bistrimethylsilylacetamid ca. 1 h auf 40°C erwärmt. Man verfolgt die Reaktion im Dünnschichtchromatogramm. Nach beendeter Umsetzung setz man zunächst 0.2 ml Eisessig zu , rührt 30 min und engt ein. Der Rückstandwird mit 20 ml einer Mischung von Isopropanol / 2N Schwefelsäure = 10:1 versetzt und 1 Std. gerührt. Nach Zugabe einer Spatelspitze festem Natriumhydrogencarbonat engt man erneut i. Vak. ein, nimmt mit Ethylacetat auf, wäscht die org. Phase mit Wasser , trocknet und reinigt nach Entfernen des Lösemittels den Rückstand durch Säulenchromatographie (SiO₂, CH₂Cl₂/Methanol = 100: 1). Man erhält das Produkt mit dem Molekulargewicht 418.45 (C₂₅H₂₀F₂N₂O₂); MS (DCI+): 419 (M+H⁺).
c) 4-{1-(4-Fluor-phenyl)-3-[3-(4-fluor-phenyl)-3-hydroxy-propyl]-4-oxo-azetidin-2-yl}-N- hydroxy-benzamidin (3):
   Zu einer Lösung aus 200 mg {1-(4-Fluor-phenyl)-3-[3-(4-fluor-phenyl)-3-hydroxypropyl]-4-oxo-azetidin-2-yl}- benzonitril, 0.45 ml Triethylamin in 15 ml Isopropanol wird 199 mg Hydroxylammoniumhydrochlorid gegeben und 12 h bei Raumtemperatur gerührt. Die Reaktionslösung wird zweimal mit Essigsäureethylester/Wasser extrahiert. Die organischen Phasen werden über Magnesiumsulfat getrocknet und eingeengt. Man erhält das Produkt mit einem Molekulargewicht von 451.48 (C₂₅H₂₃F₂N₃O₃); MS (ESI) 452.10 (M + H⁺)

### Beispiel II

### 4-{1-(4-Fluor-phenyl)-3-[3-(4-fluor-phenyl)-3-hydroxy-propyl]-4-oxo-azetidin-2-yl}-benzamidin (4):

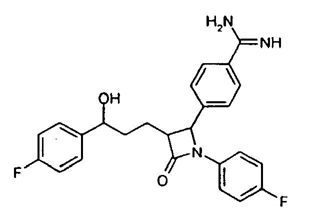

100 mg 4-{1-(4-Fluor-phenyl)-3-[3-(4-fluor-phenyl)-3-hydroxy-propyl]-4-oxo-azetidin-2-yl}-N- hydroxy-benzamidin (3) werden in 100 ml Tetrahydrofuran gelöst und mit 2 ml konz. Ammoniak über Raney-Nickel 30 Std bei 75 bar Wasserstoff und 25°C hydriert. Nach Zugabe von Magnesiumsulfat wird die Reaktionslösung filtriert. Das Filtrat wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 435.48 (C₂₅H₂₃F₂N₃O₂); MS (ESI) 436.18 (M+H⁺)

### Beispiel III

### 4-[3-[3-(4-Fluor-phenyl)-3-hydroxy-propyl]-2-(4-methoxy-phenyl)-4-oxo-azetidin-1-yl]-N-hydroxy-benzamidin (9):

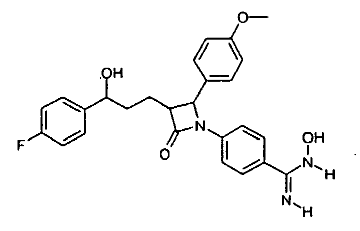

a) 3-[5-(tert-Butyl-dimethyl-silanyloxy)-5-(4-fluor-phenyl)-pentanoyl]-4-phenyl-oxazolidin-2-on (5) 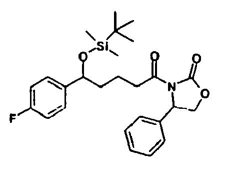
   27 g 3-[5-(4-Fluor-phenyl)-5-hydroxy-pentanoyl]-4-phenyl-oxazolidin-2-on werden mit 13,6 g Tert.-Butyl-Dimethylsilylchlorid und 10,2 g Imidazol in 36 ml Dimethylformamid gelöst und 90 min. bei 60°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Man erhält 3-[5-(tert-Butyl-dimethyl-silanyloxy)-5-(4-fluor-phenyl)-pentanoyl]-4-phenyl- oxazolidin-2-on (5) mit dem Molekulargewicht 471,65 (C₂₆H₃₄FNO₄Si); MS (ESI): 340.28 (MH⁺- HOSi(CH₃)₂C(CH₃)₃).
b) 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril (6)
   16,2 g 3-[5-(tert-Butyl-dimethyl-silanyloxy)-5-(4-fluor-phenyl)-pentanoyl]-4-phenyl-oxazolidin-2-on werden in 350 ml Dichlormethan gelöst. Die Lösung wird mit 19,8 ml Hünig Base und mit 10,14 g 4-[(4-Methoxy-phenylimino)-methyl]-benzonitril versetzt und auf-10°C gekühlt. Zur gekühlten Lösung fügt man 8,52 ml Trimethylsilyltriflat hinzu und rührt 30 min. bei -10°C. Die Lösung wird nun auf-30°C abgekühlt, und es werden 44 ml Titantetrachloridlösung zugegeben. Die Reaktionsmischung wird 2 h bei -30 bis-40°C gerührt. Danach lässt man die Lösung sich auf Raumtemperatur erwärmen, wäscht die Reaktionslösung nacheinander mit 200 ml 2N Schwefelsäure, 300 ml 20%iger Natriumhydrogensulfitllösung und ges. Kochsalzlösung. Die organische Phase wird über Magnesiumsulfat getrocknet, im Vakuum eingeengt und der Rückstand wird über Kieselgel mit n-Heptan/Essigsäureethylester 3/1 gereinigt.
   Man erhält 4-[5-(tert-Butyl-dimethyl-silanyloxy)-5-(4-fluor-phenyl)-1-(4-methoxyphenyl)-2-(2-oxo- 4-phenyl-oxazolidin-3-carbonyl)-pentylamino]-benzonitril (6) mit dem Molekulargewicht 707,93 (C₄₁H₄₆FN₃O₅Si); MS (ESI): 590.51 (MH⁺- C₇H₅N₂).
c) 4-[3-[3-(tert-Butyl-dimethyl-silanyloxy)-3-(4-fluor-phenyl)-propyl]-2-(4-methoxyphenyl)-4-oxo-azetidin-1-yl]-benzonitril (7)
   13,2 g 4-[5-(tert-Butyl-dimethyl-silanyloxy)-5-(4-fluor-phenyl)-1-(4-methoxy-phenyl)-2-(2-oxo-4-phenyl-oxazolidin-3-carbonyl)-pentylamino]-benzonitril werden in 380 ml Methyl-tert.-Butylether gelöst, mit 18,6 ml N,O-Bis(trimethylsilyl)-acetamid und 1,86 ml einer 1 M Lösung von Tetrabutylammoniumfluorid in Tetrahydrofuran versetzt und 2 h bei Raumtemperatur gerührt. Nach Beendigung der Reaktion fügt man 10 ml Essigsäure zu, engt die Reaktionsmischung im Vakuum ein und reinigt den Rückstand über Kieselgel mit Toluol/Essigsäureethylester 50/1. Man erhält 4-[3-[3-(tert-Butyl-dimethyl-silanyloxy)-3-(4-fluor-phenyl)-propyl]-2-(4-methoxy- phenyl)-4-oxo-azetidin-1-yl]-benzonitril (7) mit dem Molekulargewicht 544,75 (C₃₂H₃₇FN₂O₃Si); MS (ESI): 545.56 (M+H⁺).
d) 4-[3-[3-(4-Fluor-phenyl)-3-hydroxy-propyl]-2-(4-methoxy-phenyl)-4-oxo-azetidin-1-yl]- benzonitril (8)
   3.5 g 4-[3-[3-(tert-Butyl-dimethyl-silanyloxy)-3-(4-fluor-phenyl)-propyl]-2-(4-methoxyphenyl)-4-oxo-azetidin-1-yl]-benzonitril werden in 65 ml Tetrahydrofuran gelöst, mit 0,74 ml Essigsäure und 8,03 ml einer 1 M Lösung von Tetrabutylammoniumfluorid in Tetrahydrofuran versetzt und 2 h bei Raumtemperatur gerührt. Danach werden 4,82 ml der Tetrabutylammoniumfluorid-Lösung nachgegeben und weitere 3 h bei Rückflusstemperatur gerührt. Die abgekühlte Reaktionsmischung wird im Vakuum eingeengt, und der Rückstand wird chromatographisch über Kieselgel mit n-Heptan/Essigsäureethylester 2/1 gereinigt. Man erhält 4-[3-[3-(4-Fluor-phenyl)-3-hydroxy-propyl]-2-(4-methoxy-phenyl)-4-oxo-azetidin-1-yl]- benzonitril (8) mit dem Molekulargewicht 430,48 (C₂₆H₂₃FN₂O₃); MS (ESI): 431.24 (M+H⁺).
e) 4-[3-[3-(4-Fluor-phenyl)-3-hydroxy-propyl]-2-(4-methoxy-phenyl)-4-oxo-azetidin-1-yl]-N- hydroxy-benzamidin (9):
   Zu einer Lösung aus 200 mg 4-[3-[3-(4-Fluor-phenyl)-3-hydroxy-propyl]-2-(4-methoxyphenyl)-4-oxo-azetidin-1-yl]- benzonitril (8), 0.45 ml Triethylamin in 15 ml Isopropanol wird 199 mg Hydroxylammoniumhydrochlorid gegeben und 12 h bei Raumtemperatur gerührt. Die Reaktionslösung zweimal mit Essigsäureethylester/Wasser extrahiert. Die organischen Phasen werden über Magnesiumsulfat getrocknet und eingeengt. Man erhält das Produkt mit einem Molekulargewicht von 463.51 (C₂₆H₂₆F₁N₃O₄); MS (ESI) 464.19 (M+H⁺).

### Beispiel IV

### 4-[3-[3-(4-Fluor-phenyl)-3-hydroxy-propyl]-2-(4-methoxy-phenyl)-4-oxo-azetidin-1-yl]-benzamidin (10):

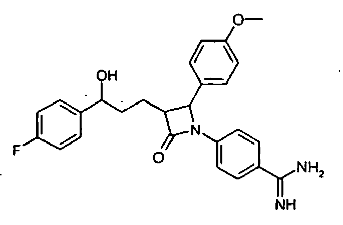

40 mg 4-[3-[3-(4-Fluor-phenyl)-3-hydroxy-propyl]-2-(4-mettioxy-phenyl)-4-oxo-azetidin-1-yl]-N- hydroxy-benzamidin werden in 10 ml Tetrahydrofuran gelöst und mit 1 ml konz. Ammoniak über Raney-Nickel 6.5 h bei 25°C hydriert. Nach Zugabe von Magnesiumsulfat wird die Reaktionslösung filtriert. Das Filtrat wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 447.51 (C₂₆H₂₆F₁N₃O₃); MS (ESI) 448.20 (M + H⁺)

### Beispiel V

### 4-{2-(4-Fluor-phenyl)-3-[3-(4-fluor-phenyl)-3-hydroxy-propyl]-4-cxo-azetidin-1-yl}-N-hydroxy-benzamidin (12):

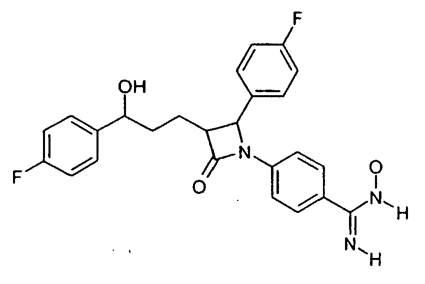

a) 4-[3-[3-(4-Fluor-phenyl)-3-hydroxy-propyl]-2-(4-fluor-phenyl)-4-oxo-azetidin-1-yl]- benzonitril (11):
   Die Verbindung (11) wird analog der Verbindung des Beispiels IIId hergestellt, mit dem Unterschied, dass anstelle von 4-[(4-Methoxy-phenylimino)-methyl]-benzonitril 4-[(4-Fluor-benzyliden)-amino]-benzonitril eingesetzt wird.
b) 4-{2-(4-Fluor-phenyl)-3-[3-(4-fluor-phenyl)-3-hydroxy-propyl]-4-oxo-azetidin-1-yl}-N- hydroxy-benzamidin (12):
   Zu einer Lösung aus 280 mg 4-[3-[3-(4-Fluor-phenyl)-3-hydroxy-propyl]-2-(4-fluorphenyl)-4-oxo-azetidin-1-yl]- benzonitril (11), 0.65 ml Triethylamin in 15 ml Isopropanol wird 279 mg Hydroxylammoniumhydrochlorid gegeben und 12 h bei Raumtemperatur gerührt. Die Reaktionslösung zweimal mit Essigsäureethylester/Wasser extrahiert. Die organischen Phasen werden über Magnesiumsulfat getrocknet und eingeengt. Man erhält das Produkt mit einem Molekulargewicht von 451.48 (C₂₅H₂₃F₂N₃O₃); MS (ESl) 452.10 (M + H⁺).

### Beispiel VI

### 4-{2-(4-Fluor-phenyl)-3-[3-(4-fluor-phenyl)-3-hydroxy-propyl]-4-oxo-azetidin-1-yl}-benzamidin (13):

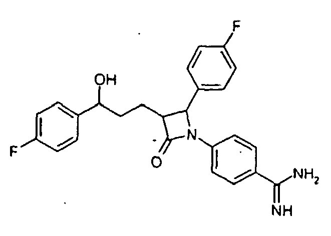

290 mg 4-{2-(4-Fluor-phenyl)-3-[3-(4-fluor-phenyl)-3-hydroxy-propyl]-4-oxo-azetidin-1-yl}-N- hydroxy-benzamidine (12) werden in 15 ml Tetrahydrofuran gelöst und mit 1.5 ml konz. Ammoniak über Raney-Nickel 6.5 h bei 25°C hydriert. Nach Zugabe von Magnesiumsulfat wird die Reaktionslösung filtriert. Das Filtrat wird eingeengt. Es erhält das Produkt mit einem Molekulargewicht von 435.48 (C₂₅H₂₃F₂N₃O₂); MS (ESI) 436.18 (M+H⁺)

### Beispiel VII

### 1-[5-(4-{1-(4-Fluor-phenyl)-3-[3-(4-fluor-phenyl)-3-hydroxy-propyl]-4-oxo-azetidin-2-yl}-benzylcarbamoyl)-pentyl]-4-aza-1-azonia-bicyclo[2.2.2]octan; trifluor-acetate (15):

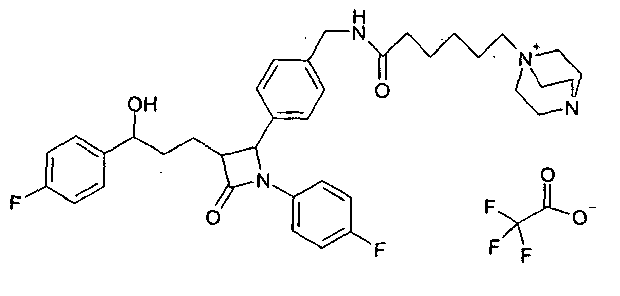

a) 1-(5-Carboxy-pentyl)-4-aza-1-azonia-bicyclo[2.2.2]octan; bromid (14):
   Zu einer Lösung von 1.5 g 1,4-Diaza-bicyclo[2.2.2]octan in 10 ml Dimethylsulfoxid werden bei 70°C 1.0 g 6-Bromhexansäure in 5 ml Dimethylsulfoxid gegeben. Nach 1 h werden 100 ml Wasser zugegeben und gefriergetrocknet. Der Rückstand wird mit Aceton digeriert. Der Rückstand enthält das Produkt mit einem Molekulargewicht von 227.33 (Kation: C₁₂H₂₃N₂O₂⁺); MS (ESI) 227.1 (M⁺).
b) 1-[5-(4-{1-(4-Fluor-phenyl)-3-[3-(4-fluor-phenyl)-3-hydroxy-propyl]-4-oxo-azetidin-2- yl}-benzylcarbamoyl)-pentyl]-4-aza-1-azonia-bicyclo[2.2.2]octan; trifluoracetate (15):
   Zu einer Lösung aus 76 mg 1-(5-Carboxy-pentyl)-4-aza-1-azonia-bicyclo[2.2.2]octan bromide (14), 64 µl Diisopropylcarbodiimid, 56 mg Hydroxybenzotriazol in 2 ml Dimethylformamid wird eine Lösung aus 70 mg 4-(4-Aminomethyl-phenyl)-1-(4-fluorphenyl)-3-[3-(4-fluor-phenyl)-3-hydroxy-propyl]- azetidin-2-on, 23 µl Triethylamin in 0.5 ml Dimethylformamid gegegeben und 12 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1 % Trifluoressigsäure) = 80/20 -> 10/90) getrennt. Man erhält das Produkt mit einem Molekulargewicht von 631.79 (Kation: C₃₇H₄₅F₂N₄O₃); MS (ESI) 631.34 (M⁺)

### Beispiel VIII

### 1-[5-(4-{3-[3-Hydroxy-3-phenyl-propyl]-2-oxo-4-phenyl-azetidin-1-yl}-benzylcarbamoyl)-pentyl]-4-aza-1-azonia-bicyclo[2.2.2]octan; trifluoracetat (17):

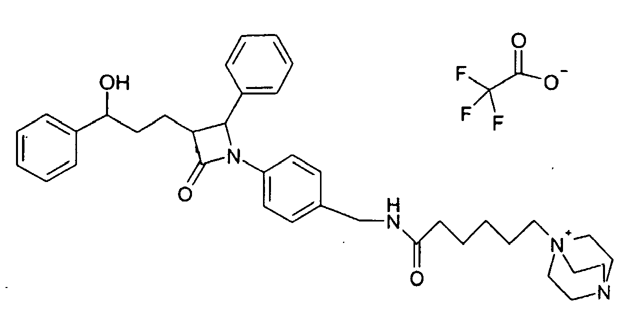

a) 1-(4-Aminomethyl-phenyl)-3-[3-hydroxy-3-phenyl-propyl]-4-phenyl-azetidin-2- on (16):
   Die Verbindung (16) wird wie in Beispiel IIIa-d beschrieben hergestellt, mit dem Unterschied, dass anstelle von 4-[(4-Methoxy-phenylimino)-methyl]-benzonitril 4-(Benzyliden-amino)-benzonitril und anstelle von 3-[5-(4-Fluor-phenyl)-5-hydroxy-pentanoyl]-4-phenyl-oxazolidin-2-on 3-[5-Phenyl)-5-hydroxy-pentanoyl]-4-phenyl-oxazolidin-2-on eingesetzt wird und dass das Produkt IIId der Reduktion mit Raney Nickel unterzogen wird.
b) 1-[5-(4-{3-[3-Hydroxy-3-phenyl-propyl]-2-oxo-4-phenyl-azetidin-1-yl}-benzylcarbamoyl)-pentyl]-4-aza-1-azonia-bicyclo[2.2.2]octan; trifluoracetate (17):
   Die Synthese erfolgt analog Beispiel Vllb ausgehend von 60 mg 1-(4-Aminomethyl-phenyl)-3-[3-(4-fluor-phenyl)-3-hydroxy-propyl]-4-phenyl-azetidin-2- on. Man erhält das Produkt mit einem Molekulargewicht von 595.81 (Kation: C₃₇H₄₇N₄O₃); MS (ESI) 595.36 (M⁺).

### Beispiel IX

### 1-[11-(4-{1-(4-Fluor-phenyl)-3-[3-(4-fluor-phenyl)-3-hydroxy-propyl]-4-oxo-azetidin-2-yl}-benzylcarbamoyl)-undecyl]-4-aza-1-azonia-bicyclo[2.2.2]octan; trifluoracetat (19):

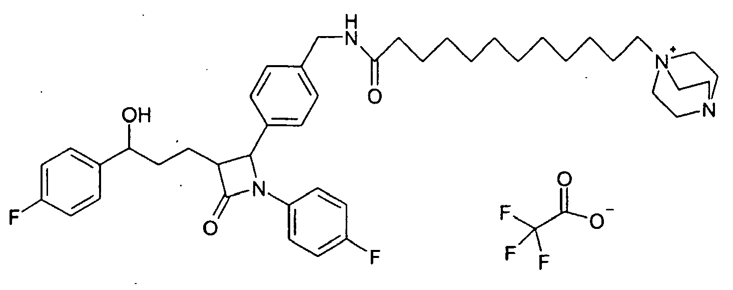

a) 1-(11-Carboxy-undecyl)-4-aza-1-azonia-bicydo[2.2.2]octan; bromid (18):
   Die Synthese erfolgt analog zu Beispiel Vlla ausgehend von 495 mg 12-Bromdodecansäure. Man erhält das Produkt mit einem Molekulargewicht von 311.49 (Kation: C₁₈H₃₅N₂O)₂⁺); MS (ESI) 311.2 (M⁺).
b) 1-[11-(4-{1-(4-Fluor-phenyl)-3-[3-(4-fluor-phenyl)-3-hydroxy-propyl]-4-oxo-azetidin-2- yl}-benzylcarbamoyl)-undecyl]-4-aza-1-azonia-bicyclo[2.2.2]octan; trifluoracetat (19):
   Die Synthese erfolgt analog zu Beispiel Vllb. Man erhält das Produkt mit einem Molekulargewicht von 715.96 (Kation: C₄₃H₅₇F₂N₄O₃); MS (ESI) 715.43 (M⁺)

### Beispiel X

### 1-(11-{4-[3-(3-Hydroxy-3-phenyl-propyl)-2-oxo-4-phenyl-azetidin-1-yl]-benzylcarbamoyl}- undecyl)-4-aza-1-azonia-bicyclo[2.2.2]octan; trifluoracetat (20):

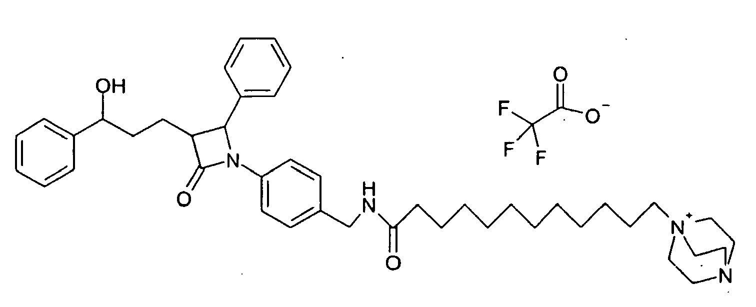

Die Synthese erfolgt analog Beispiel IXb ausgehend von 74 mg 1-(4-Aminomethyl-phenyl)-3-[3-(4-fluor-phenyl)-3-hydroxy-propyl]-4-phenyl-azetidin-2- on. Man erhält das Produkt mit einem Molekulargewicht von 679.97 (Kation: C₄₃H₅₉N₄O₃); MS (ESI) 679.50 (M⁺).

### Beispiel XI

### 1-(11-{4-[3-[3-(4-Fluor-phenyl)-3-hydroxy-propyl]-2-(4-methoxy-phenyl)-4-oxo-azetidin-1-yl]-benzylcarbamoyl}-undecyl)-4-aza-1-azonia-bicyclo[2.2.2]octan; trifluoracetat (21):

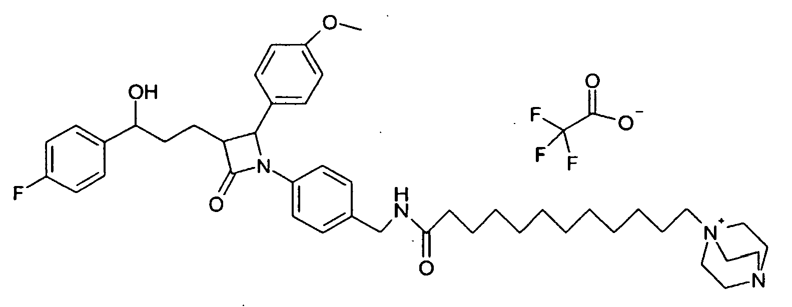

a)
   Zu einer Lösung aus 70 mg 12-Brom-dodecansäure, 50 mg EDC, 40 mg Hydroxybenzotriazol in 3 ml Dimethylformamid wird eine Lösung aus 50 mg 1-(4-Aminomethyl-phenyl)-3-[3-(4-fluor-phenyl)-3-hydroxy-propyl]-4-(4-methoxy-phenyl)-azetidin-2-on, 25 µl Triethylamin in 1 ml Dimethylformamid gegegeben und 1 h bei Raumtemperatur gerührt. Die Reaktionslösung wird mit Essigester verdünnt und dreimal mit wässriger Natriumchloridlösung gewaschen. Die organische Phase wird über Kieselgel filtriert, eingeengt und über Flashchromatographie getrennt. Man erhält das Alkylbromid (72 mg) mit einem Molekulargewicht von 695.72 (C₃₈H₄₈BrFN₂O₄); MS (ESI) 695.4 (M + H⁺).
b)
   72 mg des vorher hergestellten Alkylbromids werden zusammen mit 100 mg DABCO in 4 ml Toluol 20 Stunden bei 100 °C gerührt.Die Reaktionslösung wird eingeengt und über HPLC (Knauer Eurospher-100-10-C18, Wasser (0.1 %
   Trifluoressigsäure)/Acetonitril (0.1 % Trifluoressigsäure) = 80/20 -> 10/90) getrennt. Man erhält das Produkt mit einem Molekulargewicht von 727.99 (Kation: C₄₄H₆₀F₁N₄O₄): MS (ESI) 727.5 (M⁺)

### Beispiel XII

### N-{4-[3-(3-Hydroxy-3-phenyl-propyl)-2-oxo-4-phenyl-azetidin-1-yl]-benzyl}-N',N"-diisopropyl-guanidin (22)

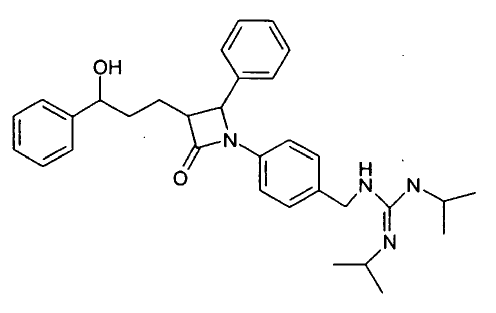

Eine Lösung aus 76 mg 1-(4-Aminomethyl-phenyl)-3-[3-hydroxy-3-phenyl-propyl]-4-phenyl-azetidin-2- on, 64 µl Diisopropylcarbodiimid, 56 mg Hydroxybenzotriazol und 23 µl Trietylamin in 2 ml Dimethylformamid wird 22 h bei Raumtemperatur gerührt. Die Reaktionslösung wird eingeengt und über HPLC(Knauer Eurospher-100-10-C18, Wasser (0.1 % Trifluoressigsäure)/Acetonitril (0.1% Trifluoressigsäure) = 80/20 -> 10/90) getrennt. Man erhält das Produkt mit einem Molekulargewicht von 512.70 (C₃₂H₄₀N₄O₂); MS (ESI) 513.4 (M + H⁺).

### Beispiel XIII

### N-(3-{1-(4-Fluor-phenyl)-3-[3-(4-fluor-phenyl)-3-hydroxy-propyl]-4-oxo-azetidin-2-yl}-benzyl)-N',N"-diisopropyl-guanidin (23)

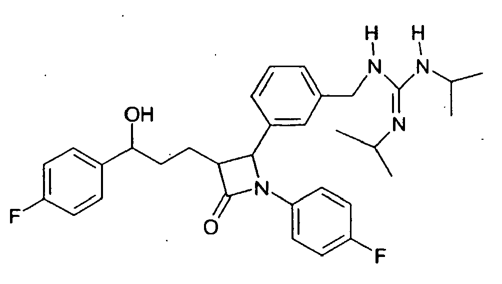

Die Synthese erfolgt analog Beispiel XII ausgehend von 60 mg 4-(4-Aminomethyl-phenyl)-1-(4-fluor-phenyl)-3-[3-(4-fluor-phenyl)-3-hydroxy-propyl]- azetidin-2-on. Man erhält das Produkt mit einem Molekulargewicht von 548.68 (C₃₂H₃₈F₂N₄O₂); MS (ESI) 549.4 (M + H⁺).
Tabelle 1: Verbindungen der Formel I

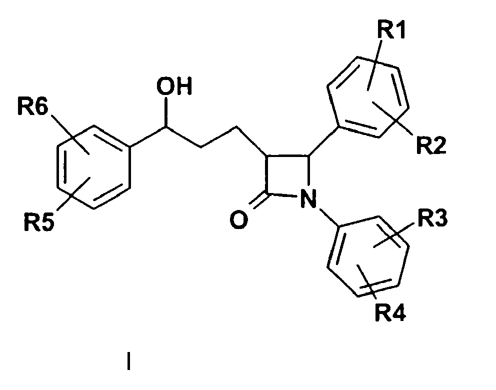

| **Bsp.** | **R1, R2** | **R3, R4** | **R5, R6** | **Salz** | **Molekul argewicht der freien Base bzw. Säure (berech net)** | **Molekul argewicht (gefund en)** |
|---|---|---|---|---|---|---|
| XIV | para-O-CH₃, H | 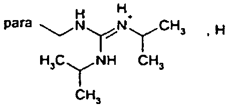 | para-F, H | CF₃C OO⁻ | 560,72 | 561,23 (MH⁺) |
| XV | para-O-CH₃, H | 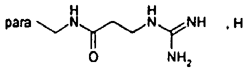 | para-F, H | - | 547,63 | 548,33 (MH⁺) |
| XVI | 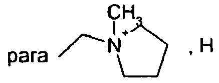 | H, H | para-F, H | I⁻ | 473,26 | 473,3 (M⁺) |
| XVII | 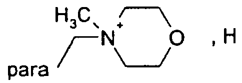 | H, H | para-F, H | I⁻ | 489,26 | 489,3 (M⁺) |
| XVIII | 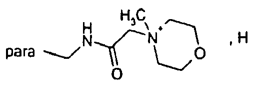 | 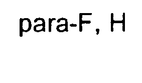 | para-F, H | I⁻ | 564,27 | 564,3 (M⁺) |
| XIX | para-O-CH₃, H | 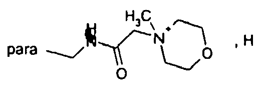 | para-F, H | I⁻ | 576,29 | 576,3 (M⁺) |
| XXI | 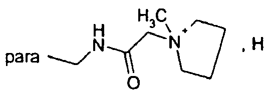 | para-F, H | para-F, H | I⁻ | 548,27 | 548,3 (M⁺) |
| XXI | para-O-CH₃, H | 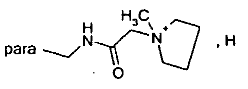 | para-F, H | I⁻ | 560,29 | 560,3 (M⁺) |
| XXII | para-O-CH₃, H | 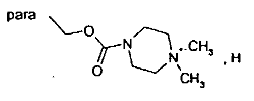 | para-F, H | I⁻ | 576,29 | 576,3 (M⁺) |
| XXIII | para-O-CH₃, H | 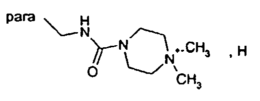 | para-F, H | I⁻ | 575,30 | 575,4 (M⁺) |
| XXIV | H, H | 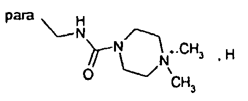 | H, H | I⁻ | 527,30 | 527,3 (M⁺) |
| XXV | 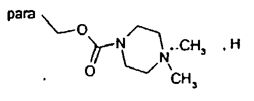 | para-F, H | para-F, H | I⁻ | 564,27 | 564,3 (M⁺) |
| XXVI | para-O-CH₃, H | 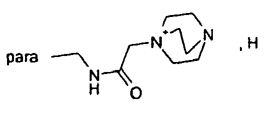 | para-F, H | I⁻ | 587,30 | 587,4 (M⁺) |
| XXVII | 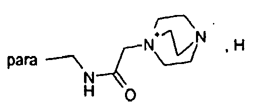 | para-F, H | para-F, H | I⁻ | 575,28 | 575,3 (M⁺) |
| XXVIII | 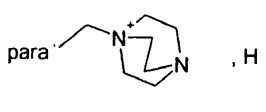 | para-F, H | para-F, H | CF₃C OO⁻ | 518,26 | 518,3 (M⁺) |
| XXIX | 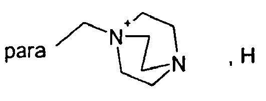 | H, H | para-F, H | Br⁻ | 500,27 | 500,3 (M⁺) |
| XXX | para-O-CH₃, H | 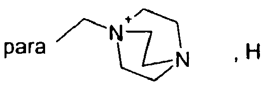 | para-F, H | Br⁻ | 530,28 | 530,3 (M⁺) |
| XXXI | 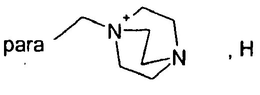 | para-F, H | H, H | Br⁻ | 500,27 | 500,3 (M⁺) |
| XXXII | 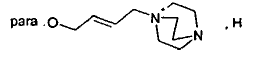 | para-F, H | para-F, H | Br⁻ | 574,29 | 574,3 (M⁺) |
| XXXIII | para-O-CH₃, H | 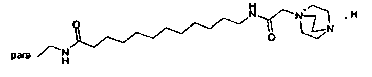 | para-F, H | Br⁻ | 784,48 | 784,5 (M⁺) |
| XXXIV | 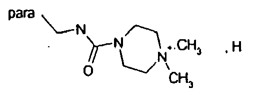 | para-F, H | para-F, H | I⁻ | 563,28 | 563,3 (M⁺) |

Die erfindungsgemäßen Verbindungen der Formel I wurden mit der nachfolgend beschriebenen Methode auf ihre Wirkung geprüft:

### Beeinflussung der Cholesterolabsorption + ³H- Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia- Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen ) oder geeignetem Vehikel angesetzt.
Das Applikationsvolumen der Testsubstanz beträgt 0,5 ml /Maus. Die Testsubstanz wird unmittelbar vor der Testmahlzeit (Intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C-Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H2O und ¹⁴C- CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Die folgenden ED₅₀-Werte belegen die Aktivität der erfindungsgemäßen

### Verbindungen der Formel 1

| Beispiel Nr. | ED₅₀ (Leber) [mg/Maus] |
|---|---|
| III | 0.1 |
| VII | 0.1 |
| IX | 0.1 |
| X | < 1.0 |
| XI | 0.3 |
| XV | 0.3 |
| XVIII | 0.3 |
| XIX | 0.3 |
| XXI | 0.1 |
| XXII | 0.3 |
| XXV | 0.1 |
| XXVI | 0.03 |
| XXXII | 0.3 |
| XXXIV | 0.3 |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel I wurde Caco-Zellmodell geprüft (A.R. Hilgers et al., Caco-2 cell monolayers as a model for drug transport across the intestinal mucosa, Pharm. Res. 1990 , 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen.

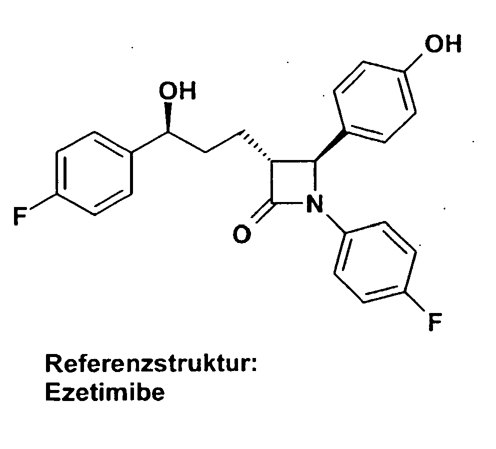

## Patentansprüche

1. Verbindungen der Formel 1, 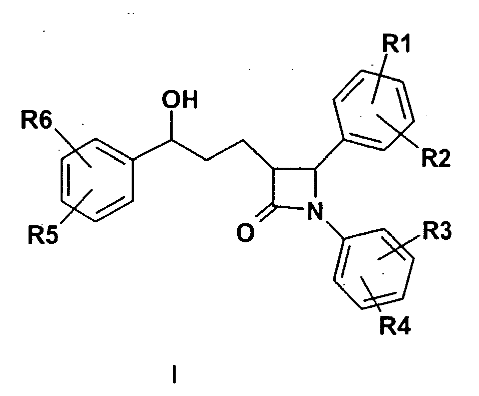 worin bedeuten
R1, R2, R3, R4, R5, R6 unabhängig voneinander (C₀-C₃₀)-Alkylen-(LAG)ₙ, wobei n = 1 - 5 sein kann und wobei ein oder mehrere C-Atome des Alkylenrests durch -S(O)ₙ-, mit n = 0 - 2, -O-, -(C=O)-, -(C=S)-, -CH=CH-, -C≡C-, -N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
(LAG)ₙ mono-, bi- oder tricyclischer Trialkylammonium-Rest, mono-, bi- oder tricyclischer Trialkylammoniumalkyl-Rest, -(CH₂)₀₋₁₀-C(=NH)(NH₂), - (CH₂)₀₋₁₀-C(=NH)(NHOH) oder -NR7-C(=NR8)(NR9R10);
R7, R8, R9 und R10 unabhängig voneinander H, (C₁-C₆)-Alkyl, (C₁-C₆)-Alkyl-Phenyl, Phenyl, (C₃-C₈)-Cycloalkyl);
n 1, 2, 3, 4, 5;
wobei immer mindestens einer der Reste R1 bis R6 die Bedeutung
(C₀-C₃₀)-Alkylen-(LAG)ₙ, wobei n = 1 - 5 ist und worin ein oder mehrere C-Atome des Alkylenrests durch -S(O)ₙ-, mit n = 0 - 2, -O-, -(C=O)-, -(C=S)-, -CH=CH-, -C=C-, - N((C₁-C₆)-Alkyl)-, -N(Phenyl)-, -N((C₁-C₆)-Alkyl-Phenyl)-, -N(CO-(CH₂)₁₋₁₀-COOH)- oder -NH- ersetzt sein können,
besitzen muß,
sowie deren pharmazeutisch verträglichen Salze.

2. Verbindungen der Formel 1, gemäß Anspruch 1, **dadurch gekennzeichnet, daß** darin bedeuten
R2, R4, R5, R6 unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1, R3 unabhängig voneinander (C₀-C₃₀)-Alkylen-(LAG) und wobei ein oder mehrere C-Atome des Alkylenrests durch -O-, -(C=O)-, -N(CH₃)- oder - NH- ersetzt sein können;
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
(LAG) mono-, bi- oder tricyclischer Trialkylammonium-Rest, mono-, bi- oder tricyclischer Trialkylammoniumalkyl-Rest, -(CH₂)₀₋₁₀-C(=NH)(NH₂), - (CH₂)₀₋₁₀-C(=NH)(NHOH) oder -NR7-C(=NR8)(NR9R10);
R7, R8, R9 und R10 unabhängig voneinander H, (C₁-C₆)-Alkyl, (C₁-C₆)-Alkyl-Phenyl, Phenyl, (C₃-C₈)-Cycloalkyl);
wobei immer mindestens einer der Reste R1 oder R3 die Bedeutung (C₀-C₃₀)-Alkylen-(LAG) und worin ein oder mehrere C-Atome des Alkylenrests durch - O-, -(C=O)-, -N(CH₃)- oder -NH- ersetzt sein können;
besitzen muß,
sowie deren pharmazeutisch verträglichen Salze.

3. Verbindungen der Formel I, gemäß Anspruch 1 oder 2, **dadurch gekennzeichnet, daß** darin bedeuten
R2, R4, R5, R6 unabhängig voneinander H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R1, R3 unabhängig voneinander -(CH₂)₀₋₁-Y-W-(C₀-C₂₅)-Alkylen-Y'-W'-(LAG), worin ein oder mehrere C-Atome des Alkylenrests durch -O- ersetzt sein können
H, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
C(=NH)(NH₂), PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
Y, W, Y' W' unabhängig voneinander NH, NCH₃, C=O, O, eine Bindung oder S(O)ₙ, mit n = 0 - 2;
oder Y-W oder Y'-W' jeweils zusammen genommen eine Bindung.
(LAG) mono-, bi- oder tricyclischer Trialkylammonium-Rest, mono-, bi- oder tricyclischer Trialkylammoniumalkyl-Rest, -(CH₂)₀₋₁₀-C(=NH)(NH₂), - (CH₂)₀₋₁₀-C(=NH)(NHOH) oder -NR7-C(=NR8)(NR9R10);
R7, R8, R9 und R10 unabhängig voneinander H, (C₁-C₆)-Alkyl, (C₁-C₆)-Alkyl-Phenyl, Phenyl, (C₃-C₈)-Cycloalkyl);
wobei immer mindestens einer der Reste R1 oder R3 die Bedeutung
-(CH₂)₀₋₁-Y-W-(C₀-C₂₅)-Alkylen-Y'-W'-(LAG), worin ein oder mehrere C-Atome des Alkylenrests durch -O- ersetzt sein können;
besitzen muß,
sowie deren pharmazeutisch verträglichen Salze.

4. Verbindungen der Formel I gemäß einem oder mehreren der Ansprüche 1 bis 3, **dadurch gekennzeichnet, daß** darin bedeuten
LAG tricyclischer Trialkylammoniumalkyl-Rest,
sowie deren pharmazeutisch verträglichen Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und mindestens einen weiteren Wirkstoff.

7. Arzneimittel, gemäß Anspruch 6, **dadurch gekennzeichnet, daß** es als weiteren Wirkstoff eine oder mehrere Verbindungen, die den Lipidstoffwechsel normalisieren, enthält.

8. Arzneimittel, gemäß Anspruch 6 oder 7, **dadurch gekennzeichnet, daß** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren,
Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren,
Lipoprotein(a) antagonisten, Lipase Inhibitoren, Insuline, Sulphonylharnstoffe,
Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, *β*3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen,
5HT-Agonisten, Bombesin-Agonisten, Galanin-Antagonisten, Wachstumshormone,
Wachstumshormon freisetzende Verbindungen, TRH-Agonisten, entkoppelnde Protein 2- oder 3-Modulatoren, Leptinagonisten, DA-Agonisten (Bromocriptin, Doprexin),
Lipase/Amylase-Inhibitoren, PPAR-Modulatoren, RXR-Modulatoren oder TR-*β-*Agonisten oder Amphetamine enthält.

9. Verbindungen gemäß einem oder mehreren der Ansprüche 1 bis 4 zur Anwendung als Medikament zur Behandlung von Lipidstoffwechselstörungen.

10. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 4 zur Herstellung eines Medikaments zur Behandlung von Hyperlipidämie.

12. Verwendung der Verbindungen gemäß einem oder mehreren der Ansprüche 1 bis 4 zur Herstellung eines Medikaments zur Senkung des Serumcholesterinspiegels.

13. Verwendung der Verbindungen gemäß einem oder mehreren der Ansprüche 1 bis 4 zur Herstellung eines Medikaments zur Behandlung arteriosklerotischer Erscheinungen.

14. Verwendung der Verbindungen gemäß einem oder mehreren der Ansprüche 1 bis 4 zur Herstellung eines Medikaments zur Behandlung von Insulin Resistenz.

## Claims

1. A compound of the formula I, 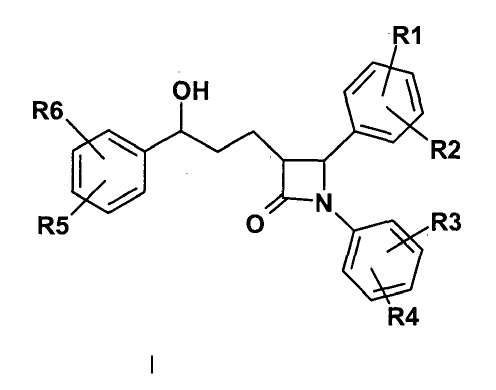 in which
R1, R2, R3, R4, R5, R6 independently of one another are (C₀-C₃₀)-alkylene-(LAG)ₙ, where n may be 1 - 5 and where one or more carbon atoms of the alkylene radical may be replaced by -S(O)ₙ-, where n = 0-2, -O-, -(C=O)-, -(C=S)-, -CH=CH-, -C≡C-, -N((C₁-C₆)-alkyl)-, -N(phenyl)-, -N((C₁-C₆)-alkyl-phenyl)- , -N(CO-(CH₂)₁₋₁₀-COOH)- or -NH-; H, F, Cl, Br, I, CF₃, NO₂, N₃, CN, COOH, COO(C₁-C₆)-alkyl, CONH₂,
CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
(LAG)ₙ is a mono-, di- or tricyclic trialkylammonium radical, a mono-, di- or tricyclic trialkylammoniumalkyl radical, -(CH₂)₀₋₁₀-C(=NH)(NH₂), -(CH₂)₀₋₁₀-C(=NH)(NHOH) or -NR7-C(=NR8)(NR9R10);
R7, R8, R9 and R10 independently of one another are H, (C₁-C₆)-alkyl, (C₁-C₆)-alkyl-phenyl, phenyl, (C₃-C₈)-cycloalkyl);
n is 1, 2, 3, 4, 5;
where in each case at least one of the radicals R1 to R6 must have the meaning (C₀-C₃₀)-alkylene-(LAG)ₙ, where n = 1 - 5 and where one or more carbon atoms of the alkylene radical may be replaced by -S(O)ₙ-, where n = 0 - 2, -O-, -(C=O)-, -C=S)-, -CH=CH-, -C≡C-, -N((C₁-C₆)-alkyl)-, -N(phenyl)-, -N((C₁-C₆)-alkyl-phenyl)-, -N(CO-(CH₂)₁₋₁₀-COOH)- or -NH-;
and its pharmaceutically acceptable salts.

2. A compound of the formula I as claimed in claim 1, wherein
R2, R4, R5, R6 independently of one another are H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1, R3 independently of one another are (C₀-C₃₀)-alkylene-(LAG) and where one or more carbon atoms of the alkylene radical may be replaced by -O-, -(C=O)-, -N(CH₃)- or -NH-,
H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
(LAG) is a mono-, di- or tricyclic trialkylammonium radical, a mono-, di- or tricyclic trialkylammoniumalkyl radical, -(CH₂)₀₋₁₀-C(=NH)(NH₂), -(CH₂)₀-₁₀-C(=NH)(NHOH) or -NR7-C(=NR8)(NR9R10);
R7, R8, R9 and R10 independently of one another are H, (C₁-C₆)-alkyl, (C₁-C₆)-alkyl-phenyl, phenyl, (C₃-C₈)-cycloalkyl;
where in each case at least one of the radicals R1 or R3 must have the meaning (C₀-C₃₀)-alkylene-(LAG), where one or more carbon atoms of the alkylene radical may be replaced by -O-, -(C=O)-, -N(CH₃)- or -NH-;
and its physiologically acceptable salts.

3. A compound of the formula I as claimed in claim 1 or 2, wherein
R2, R4, R5, R6 independently of one another are H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R1, R3 independently of one another are -(CH₂)₀₋₁-Y-W-(C₀-C₂₅)-alkylene-Y'-W'-(LAG), where one or more carbon atoms of the alkylene radical may be replaced by -O-;
H, F, Cl, Br, I, CF₃, NO₂, N₃, CN, COOH, COO(C₁-C₆)-alkyl, CONH₂, CONH(C₁-C₆)-alkyl, CON[(C₁-C₆)-alkyl]_{2,} (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
C(=NH)(NH₂), PO₃H₂, SO₃H, SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
Y, W, Y' W' independently of one another are NH, NCH₃, C=O, O, a bond or S(O)ₙ,
or Y-W or Y'-W' taken together are each a bond;where n=0-2;
(LAG) is a mono-, di- or tricyclic trialkylammonium radical, a mono-, di- or tricyclic trialkylammoniumalkyl radical, -(CH₂)₀₋₁₀-C(=NH)(NH₂), -(CH₂)₀₋₁₀-C(=NH)(NHOH) or -NR7-C(=NR8)(NR9R10);
R7, R8, R9 and R10 independently of one another are H, (C₁-C₆)-alkyl, (C₁-C₆)-alkyl-phenyl, phenyl, (C₃-C₈)-cycloalkyl);
where in each case at least one of the radicals R1 or R3 must have the meaning -(CH₂)₀₋₁-Y-W-(C₀-C₂₅)-alkylene-Y'-W'-(LAG), where one or more carbon atoms of the alkylene radical may be replaced by -O-; and its pharmaceutically acceptable salts.

4. A compound of the formula I as claimed in one or more of claims 1 to 3, wherein
LAG is a tricyclic trialkylammoniumalkyl radical,
and its pharmaceutically acceptable salts.

5. A medicament comprising one or more compounds as claimed in one or more of claims 1 to 4.

6. A medicament comprising one or more compounds as claimed in one or more of claims 1 to 4 and at least one further active compound.

7. The medicament as claimed in claim 6, comprising, as further active compound, one or more compounds which normalize lipid metabolism.

8. The medicament as claimed in claim 6 or 7, which comprises, as further active compound, one or more
antidiabetics, hypoglycemically active compounds, HMGCoA reductase inhibitors, cholesterol absorption inhibitors, PPAR gamma agonists, PPAR alpha agonists,
PPAR alpha/gamma agonists, fibrates, MTP inhibitors, bile acid absorption inhibitors,
CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active compounds which act on the ATP-dependent potassium channel of the beta cells, CART agonists, NPY agonists, MC4 agonists, orexin agonists, H3 agonists,
TNF agonists, CRF agonists, CRF BP antagonists, urocortin agonists, β3 agonists,
MSH (melanocyte-stimulating hormone) agonists, CCK agonists, serotonin-reuptake inhibitors, mixed serotonin and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptine, doprexin), lipase/amylase inhibitors, PPAR modulators,
RXR modulators or TR-β-agonists or amphetamines.

9. A compound as claimed in one or more of claims 1 to 4 for use as a medicament for the treatment of impaired lipid metabolism.

10. A process for preparing a medicament comprising one or more of the compounds as claimed in one or more of claims 1 to 4, which comprises mixing the active compound with a pharmaceutically acceptable carrier and bringing this mixture into a form suitable for administration.

11. The use of the compounds as claimed in one or more of claims 1 to 4 for preparing a medicament for treating hyperlipidemia.

12. The use of the compounds as claimed in one or more of claims 1 to 4 for preparing a medicament for lowering the serum cholesterol concentration.

13. The use of the compounds as claimed in one or more of claims 1 to 4 for preparing a medicament for treating arteriosclerotic manifestations.

14. The use of the compounds as claimed in one or more of claims 1 to 4 for preparing a medicament for treating insulin resistance.

## Revendications

1. Composés de formule I, 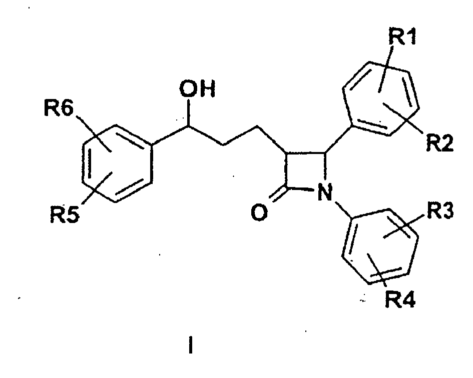 dans laquelle
R1, R2, R3, R4, R5, R6 représentent, indépendamment les uns des autres, un groupe (C₀-C₃₀)-alkylène-(LAG)ₙ, dans lequel n peut valoir de 1 à 5, et dans lequel un ou plusieurs atomes de carbone du radical alkylène peuvent être remplacés par un groupe -S(O)ₙ-, dans lequel n vaut de 0 à 2, un groupe -O-, un groupe -(C=O)-, un groupe -(C=S)-, un groupe -CH=CH-, un groupe -C≡C-, un groupe -N((C₁-C₆)-alkyl)-, un groupe -N(phényl)-, un groupe -N((C₁-C₆)-alkyl-phényl)-, un groupe -N(CO-(CH₂)₁₋₁₀-COOH)- ou un groupe -NH- ;
un atome d'hydrogène, un atome de fluor, un atome de chlore, un atome de brome, un atome d'iode, un groupe CF₃, un groupe NO₂, un groupe N₃, un groupe CN, un groupe COOH, un groupe COO (C₁-C₆) -alkyle, un groupe CONH₂, un groupe CONH (C₁-C₆)-alkyle, un groupe CON [(C₁-C₆)-alkyle]₂, un groupe (C₁-C₆)-alkyle, un groupe (C₂-C₆)-alcényle, un groupe (C₂-C₆) -alcynyle, un groupe O-(C₁-C₆)-alkyle, où, dans les radicaux alkyle, un, plusieurs ou la totalité des atomes d'hydrogène peuvent être remplacés par du fluor ;
un groupe C(=NH)(NH₂), un groupe PO₃H₂, un groupe SO₃H, un groupe SO₂-NH₂, un groupe SO₂NH (C₁-C₆)-alkyle, un groupe SO₂N[(C₁-C₆)-alkyle]₂, un groupe S-(C₁-C₆)-alkyle, un groupe S-(CH₂)ₙ-phényle, un groupe SO-(C₁-C₆)-alkyle, un groupe SO-(CH₂)ₙ-phényle, un groupe SO₂-(C₁-C₆)-alkyle, un groupe SO₂-(CH₂)ₙ-Phényle, où n peut valoir de 0 à 6 et le radical phényle peut être jusqu'à bi-substitué par un atome de fluor, un atome de chlore, un atome de brome, un groupe OH, un groupe CF₃, un groupe NO₂, un groupe CN, un groupe OCF₃, un groupe O-(C₁-C₆)-alkyle, un groupe (C₁-C₆)-alkyle, un groupe NH₂ ;
un groupe NH₂, un groupe NH-(C₁-C₆)-alkyle, un groupe N ((C₁-C₆)-alkyle)₂, un groupe NH(C₁-C₇)-acyle, un groupe phényle, un groupe O-(CH₂)ₙ-phényle, où n peut valoir de 0 à 6, où le noyau phényle peut être mono- jusqu'à tri-substitué par un atome de fluor, un atome de chlore, un atome de brome, un atome d'iode, un groupe OH, un groupe CF₃, un groupe NO₂, un groupe CN, un groupe OCF₃, un groupe 0- (C₁-C₆) -alkyle, un groupe (C₁-C₆)-alkyle, un groupe NH₂, un groupe NH (C₁-C₆)-alkyle, un groupe N ((C₁-C₆)-alkyle)₂, un groupe SO₂-CH₃, un groupe COOH, un groupe COO-(C₁-C₆)-alkyle, un groupe CONH₂ ;
(LAG)ₙ représente un radical trialkylammonium mono-, bi- ou tri-cyclique, un radical trialkyl-ammoniumalkyle mono-, bi- ou tri-cyclique, un groupe - (CH₂)₀₋₁₀-C (=NH) (NH₂), un groupe - (CH₂)₀₋₁₀-C (=NH) (NHOH) ou un groupe -NR7-C(=NR8)(NR9R10) ;
R7, R8, R9 et R10 représentent, indépendamment les uns des autres, un atome d'hydrogène, un groupe (C₁-C₆)-alkyle, un groupe (C₁-C₆)-alkyl-phényle, un groupe phényle, un groupe (C₃-C₈)-cyclo-alkyle ;
n vaut 1, 2, 3, 4, 5 ;
où au moins l'un des radicaux R1 à R6 doit toujours représenter un groupe (C₀-C₃₀)-alkylène-(LAG)ₙ, dans lequel n vaut de 1 à 5 et dans lequel un ou plusieurs atomes de carbone du radical alkylène peuvent être remplacés par un groupe -S(O)ₙ-, dans lequel n vaut de 0 à 2, un groupe -O-, un groupe -(C=O)-, un groupe -(C=S)-, un groupe -CH=CH-, un groupe -C≡C-, un groupe -N((C₁-C₆)-alkyl)-, un groupe -N (phényl) -, un groupe -N (C₁-C₆) -alkyl-phényl) -, un groupe -N(CO-(CH₂)₁₋₁₀-COOH)- ou un groupe -NH- ;
ainsi que leurs sels pharmaceutiquement acceptables.

2. Composés de formule I selon la revendication 1, **caractérisés en ce que** :
R2, R4, R5, R6 représentent, indépendamment les uns des autres, un atome d'hydrogène, un atome de fluor, un atome de chlore, un atome de brome, un atome d'iode, un groupe CF₃, un groupe NO₂, un groupe N₃, un groupe CN, un groupe COOH, un groupe COO (C₁-C₆)-alkyle, un groupe CONH₂, un groupe CONH (C₁-C₆)-alkyle, un groupe CON [(C₁-C₆)-alkyle]₂, un groupe (C₁-C₆)-alkyle, un groupe (C₂-C₆)-alcényle, un groupe (C₂-C₆)-alcynyle, un groupe O-(C₁-C₆)-alkyle, où, dans les radicaux alkyle, un, plusieurs ou la totalité des atomes d'hydrogène peuvent être remplacés par du fluor ;
un groupe C(=NH)(NH₂), un groupe PO₃H₂, un groupe SO₃H, un groupe SO₂-NH₂, un groupe SO₂NH(C₁-C₆) -alkyle, un groupe SO₂N [(C₁-C₆)-alkyle]₂, un groupe S- (C₁-C₆)-alkyle, un groupe S-(CH₂)ₙ-phényle, un groupe SO-(C₁-C₆)-alkyle, un groupe SO-(CH₂)ₙ-phényle, un groupe SO₂-(C₁-C₆)-alkyle, un groupe SO₂-(CH₂)ₙ-phényle, où n peut valoir de 0 à 6 et le radical phényle peut être jusqu'à bi-substitué par un atome de fluor, un atome de chlore, un atome de brome, un groupe OH, un groupe CF₃, un groupe NO₂, un groupe CN, un groupe OCF₃, un groupe O-(C₁-C₆)-alkyle, un groupe (C₁-C₆)-alkyle, un groupe NH₂ ;
un groupe NH₂, un groupe NH-(C₁-C₆)-alkyle, un groupe N ((C₁-C₆)-alkyle)₂, un groupe NH(C₁-C₇)-acyle, un groupe phényle, un groupe O-(CH₂)ₙ-phényle, où n peut valoir de 0 à 6, où le noyau phényle peut être mono- jusqu'à tri-substitué par un atome de fluor, un atome de chlore, un atome de brome, un atome d'iode, un groupe OH, un groupe CF₃, un groupe NO₂, un groupe CN, un groupe OCF₃, un groupe O- (C₁-C₆) -alkyle, un groupe (C₁-C₆)-alkyle, un groupe NH₂, un groupe NH (C₁-C₆) -alkyle, un groupe N ((C₁-C₆) -alkyle)₂, un groupe SO₂-CH₃, un groupe COOH, un groupe COO-(C₁-C₆)-alkyle, un groupe CONH₂;
R1, R3 représentent, indépendamment l'un de l'autre, un groupe (C₀-C₃₀) -alkylène- (LAG) et dans lequel un ou plusieurs atomes de carbone du radical alkylène peuvent être remplacés par un groupe -O-, un groupe -(C=O)-, un groupe -N(CH₃)- ou un groupe -NH-,
un atome d'hydrogène, un atome de fluor, un atome de chlore, un atome de brome, un atome d'iode, un groupe CF₃, un groupe NO₂, un groupe N₃, un groupe CN, un groupe COOH, un groupe COO(C₁-C₆)-alkyle, un groupe CONH₂, un groupe CONH (C₁-C₆) -alkyle, un groupe CON [(C₁-C₆)-alkyle]₂, un groupe (C₁-C₆)-alkyle, un groupe (C₂-C₆)-alcényle, un groupe (C₂-C₆)-alcynyle, un groupe O-(C₁-C₆)-alkyle, où, dans les radicaux alkyle, un, plusieurs ou la totalité des atomes d'hydrogène peuvent être remplacés par du fluor ;
un groupe C(=NH)(NH₂), un groupe PO₃H₂, un groupe SO₃H, un groupe SO₂-NH₂, un groupe SO₂NH (C₁-C₆)-alkyle, un groupe SO₂N [(C₁-C₆)-alkyle]₂, un groupe S-(C₁-C₆)-alkyle, un groupe S-(CH₂)ₙ-phényle, un groupe SO-(C₁-C₆)-alkyle, un groupe SO-(CH₂)ₙ-phényle, un groupe SO₂-(C₁-C₆)-alkyle, un groupe SO₂-(CH₂)ₙ-phényle, où n peut valoir de 0 à 6 et le radical phényle peut être jusqu'à bi-substitué par un atome de fluor, un atome de chlore, un atome de brome, un groupe OH, un groupe CF₃, un groupe NO₂, un groupe CN, un groupe OCF₃, un groupe O-(C₁-C₆)-alkyle, un groupe (C₁-C₆)-alkyle, un groupe NH₂ ;
un groupe NH₂, un groupe NH-(C₁-C₆)-alkyle, un groupe N ((C₁-C₆)-alkyle)₂, un groupe NH(C₁-C₇)-acyle, un groupe phényle, un groupe O-(CH₂)ₙ-phényle, où n peut valoir de 0 à 6, où le noyau phényle peut être mono- jusqu'à tri-substitué par un atome de fluor, un atome de chlore, un atome de brome, un atome d'iode, un groupe OH, un groupe CF₃, un groupe NO₂, un groupe CN, un groupe OCF₃, un groupe 0- (C₁-C₆) -alkyle, un groupe (C₁-C₆)-alkyle, un groupe NH₂, un groupe NH (C₁-C₆) -alkyle, un groupe N((C₁-C₆)-alkyle)₂, un groupe SO₂-CH₃, un groupe COOH, un groupe COO-(C₁-C₆)-alkyle, un groupe CONH₂;
(LAG) représente un radical trialkylammonium mono-, bi- ou tri-cyclique, un radical trialkyl-ammoniumalkyle mono-, bi- ou tri-cyclique, un groupe -(CH₂)₀₋₁₀-C (=NH) (NH₂), un groupe
- (CH₂)₀₋₁₀-C (=NH) (NHOH) ou un groupe -NR7-C(=NR8)(NR9R10);
R7, R8, R9 et R10 représentent, indépendamment les uns des autres, un atome d'hydrogène, un groupe (C₁-C₆)-alkyle, un groupe (C₁-C₆)-alkyl-phényle, un groupe phényle, un groupe (C₃-C₈)-cyclo-alkyle ;
où au moins l'un des radicaux R1 ou R3 doit toujours représenter un groupe (C₀-C₃₀)-alkylène-(LAG) et dans lequel un ou plusieurs atomes de carbone du radical alkylène peuvent être remplacés par un groupe -O-, un groupe -(C=O)-, un groupe -N(CH₃)- ou un groupe -NH- ;
ainsi que leurs sels pharmaceutiquement acceptables.

3. Composés de formule I selon la revendication 1 ou 2, **caractérisés en ce que** :
R2, R4, R5, R6 représentent, indépendamment les uns des autres, un atome d'hydrogène, un atome de fluor, un atome de chlore, un atome de brome, un atome d'iode, un groupe CF₃, un groupe NO₂, un groupe N₃, un groupe CN, un groupe COOH, un groupe COO(C₁-C₆)-alkyle, un groupe CONH₂, un groupe CONH(C₁-C₆)-alkyle, un groupe CON [(C₁-C₆)-alkyle]₂, un groupe (C₁-C₆) -alkyle, un groupe (C₂-C₆)-alcényle, un groupe (C₂-C₆)-alcynyle, un groupe O-(C₁-C₆) -alkyle, où, dans les radicaux alkyle, un, plusieurs ou la totalité des atomes d'hydrogène peuvent être remplacés par du fluor ;
un groupe C(=NH)(NH₂), un groupe PO₃H₂, un groupe SO₃H, un groupe SO₂-NH₂, un groupe SO₂NH (C₁-C₆)-alkyle, un groupe SO₂N[(C₁-C₆)-alkyle]₂, un groupe S-(C₁-C₆)-alkyle, un groupe S-(CH₂)ₙ-phényle, un groupe SO-(C₁-C₆)-alkyle, un groupe SO-(CH₂)ₙ-phényle, un groupe SO₂-(C₁-C₆)-alkyle, un groupe SO₂-(CH₂)ₙ-phényle, où n peut valoir de 0 à 6 et le radical phényle peut être jusqu'à bi-substitué par un atome de fluor, un atome de chlore, un atome de brome, un groupe OH, un groupe CF₃, un groupe NO₂, un groupe CN, un groupe OCF₃, un groupe O-(C₁-C₆)-alkyle, un groupe (C₁-C₆)-alkyle, un groupe NH₂ ;
un groupe NH₂, un groupe NH-(C₁-C₆)-alkyle, un groupe N((C₁-C₆)-alkyle)₂, un groupe NH(C₁-C₇)-acyle, un groupe phényle, un groupe O-(CH₂)ₙ-phényle, où n peut valoir de 0 à 6, où le noyau phényle peut être mono- jusqu'à tri-substitué par un atome de fluor, un atome de chlore, un atome de brome, un atome d'iode, un groupe OH, un groupe CF₃, un groupe NO₂, un groupe CN, un groupe OCF₃, un groupe 0- (C₁-C₆) -alkyle, un groupe (C₁-C₆)-alkyle, un groupe NH₂, un groupe NH(C₁-C₆)-alkyle, un groupe N ((C₁-C₆)-alkyle)₂, un groupe SO₂-CH₃, un groupe COOH, un groupe COO-(C₁-C₆)-alkyle, un groupe CONH₂ ;
R1, R3 représentent, indépendamment l'un de l'autre, un groupe -(CH₂)₀₋₁-Y-W-(C₀-C₂₅)-alkylène-Y'-W'-(LAG) dans lequel un ou plusieurs atomes de carbone du radical alkylène peuvent être remplacés par un groupe -O-,
un atome d'hydrogène, un atome de fluor, un atome de chlore, un atome de brome, un atome d'iode, un groupe CF₃, un groupe NO₂, un groupe N₃, un groupe CN, un groupe COOH, un groupe COO(C₁-C₆)-alkyle, un groupe CONH₂, un groupe CONH (C₁-C₆) -alkyle, un groupe CON [(C₁-C₆)-alkyle]₂, un groupe (C₁-C₆)-alkyle, un groupe (C₂-C₆)-alcényle, un groupe (C₂-C₆)-alcynyle, un groupe O-(C₁-C₆)-alkyle, où, dans les radicaux alkyle, un, plusieurs ou la totalité des atomes d'hydrogène peuvent être remplacés par du fluor ;
un groupe C(=NH)(NH₂), un groupe PO₃H₂, un groupe SO₃H, un groupe SO₂-NH₂, un groupe SO₂NH (C₁-C₆)-alkyle, un groupe SO₂N [(C₁-C₆)-alkyle]₂, un groupe S-(C₁-C₆)-alkyle, un groupe S-(CH₂)ₙ-phényle, un groupe SO-(C₁-C₆)-alkyle, un groupe SO-(CH₂)ₙ-phényle, un groupe SO₂-(C₁-C₆)-alkyle, un groupe SO₂-(CH₂)ₙ-phényle, où n peut valoir de 0 à 6 et le radical phényle peut être jusqu'à bi-substitué par un atome de fluor, un atome de chlore, un atome de brome, un groupe OH, un groupe CF₃; un groupe NO₂, un groupe CN, un groupe OCF₃, un groupe O-(C₁-C₆)-alkyle, un groupe (C₁-C₆)-alkyle, un groupe NH₂;
un groupe NH₂, un groupe NH-(C₁-C₆)-alkyle, un groupe N ((C₁-C₆)-alkyle)₂, un groupe NH(C₁-C₇)-acyle, un groupe phényle, un groupe O-(CH₂)ₙ-phényle, où n peut valoir de 0 à 6, où le noyau phényle peut être mono- jusqu'à tri-substitué par un atome de fluor, un atome de chlore, un atome de brome, un atome d'iode, un groupe OH, un groupe CF₃, un groupe NO₂, un groupe CN, un groupe OCF₃, un groupe O- (C₁-C₆) -alkyle, un groupe (C₁-C₆)-alkyle, un groupe NH₂, un groupe NH (C₁-C₆)-alkyle, un groupe N ((C₁-C₆)-alkyle)₂, un groupe SO₂-CH₃, un groupe COOH, un groupe COO-(C₁-C₆)-alkyle, un groupe CONH₂ ;
Y, W, Y', W' représentent, indépendamment les uns des autres, un groupe NH, un groupe NCH₃, un groupe C=O, un atome d'oxygène, une liaison ou un groupe S(O)ₙ, dans lequel n vaut de 0 à 2 ;
ou Y-W ou Y'-W', dans chaque cas pris conjointement, représente une liaison ;
(LAG) représente un radical trialkylammonium mono-, bi- ou tri-cyclique, un radical trialkyl-ammoniumalkyle mono-, bi- ou tri-cyclique, un groupe -(CH₂)₀₋₁₀-C(=NH) (NH₂), un groupe -(CH₂)₀₋₁₀-C(=NH) (NHOH) ou un groupe -NR7-C(=NR8)(NR9R10) ;
R7, R8, R9 et R10 représentent, indépendamment les uns des autres, un atome d'hydrogène, un groupe (C₁-C₆)-alkyle, un groupe (C₁-C₆) -alkyl-phényle, un groupe phényle, un groupe (C₃-C₈)-cyclo-alkyle ;
où au moins l'un des radicaux R1 ou R3 doit toujours représenter un groupe -(CH₂)₀₋₁-Y-W-(C₀-C₂₅)-alkylène-Y'-W'-(LAG), dans lequel un ou plusieurs atomes de carbone du radical alkylène peuvent être remplacés par un groupe -O- ;
ainsi que leurs sels pharmaceutiquement acceptables.

4. Composés de formule I selon l'une ou plusieurs des revendications 1 à 3, **caractérisés en ce que** :
LAG représente un radical trialkylammoniumalkyle tricyclique,
ainsi que leurs sels pharmaceutiquement acceptables.

5. Médicament comprenant l'un ou plusieurs des composés selon l'une ou plusieurs des revendications 1 à 4.

6. Médicament comprenant l'un ou plusieurs des composés selon l'une ou plusieurs des revendications 1 à 4 et au moins un ingrédient actif supplémentaire.

7. Médicament selon la revendication 6, **caractérisé en ce qu'**il comprend, en tant qu'ingrédient actif supplémentaire, un ou plusieurs composés qui normalisent le métabolisme lipidique.

8. Médicament selon la revendication 6 ou 7, **caractérisé en ce qu'**il comprend, en tant qu'ingrédient actif supplémentaire, un ou plusieurs agents antidiabétiques, ingrédients actifs hypoglycémiants, inhibiteurs de HMGCoA réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption d'acide biliaire, inhibiteurs de CETP, agents adsorbants polymères d'acide biliaire, inducteurs de récepteur de LDL, inhibiteurs d'ACAT, agents antioxidants, inhibiteurs de lipoprotéine lipase, inhibiteurs d'ATP-citrate-lyase, inhibiteurs de squalène synthétase, antagonistes de lipoprotéine (a), inhibiteurs de lipase, insulines, sulfonylurées, biguanides, méglitinides, thiazolidinediones, inhibiteurs de α-glucosidase, ingrédients actifs agissant sur le canal potassique ATP-dépendant des cellules bêta, agonistes de CART, agonistes de NPY, agonistes de MC4, agonistes de l'orexine, agonistes de H3, agonistes de TNF, agonistes de CRF, antagonistes de CRF BP, agonistes de l'urocortine, agonistes de β3, agonistes de MSH (hormone mélano-stimulante), agonistes de CCK, inhibiteurs du recaptage de la sérotonine, composés séro-toninergiques et noradrénergiques mixtes, agonistes de 5HT, agonistes de la bombésine, antagonistes de la galanine, hormones de croissance, composés libérant une hormone de croissance, agonistes de TRH, modulateurs de la protéine découplante 2 ou 3, agonistes de la leptine, agonistes de DA (bromocriptine, doprexine), inhibiteurs de lipase/ amylase, modulateurs de PPAR, modulateurs de RXR ou agonistes de TR-β ou amphétamines.

9. Composés selon l'une ou plusieurs des revendications 1 à 4 pour une utilisation en tant que médicament destiné au traitement de troubles du métabolisme lipidique.

10. Procédé de production d'un médicament comprenant l'un ou plusieurs des composés selon l'une ou plusieurs des revendications 1 à 4, **caractérisé en ce que** l'ingrédient actif est mélangé avec un support pharmaceutiquement approprié et ce mélange est mis sous une forme appropriée pour l'administration.

11. Utilisation des composés selon l'une ou plusieurs des revendications 1 à 4, pour la production d'un médicament destiné au traitement de l'hyper-lipidémie.

12. Utilisation des composés selon l'une ou plusieurs des revendications 1 à 4, pour la production d'un médicament destiné à réduire le taux de cholestérol sérique.

13. Utilisation des composés selon l'une ou plusieurs des revendications 1 à 4, pour la production d'un médicament destiné au traitement de manifestations artériosclérotiques.

14. Utilisation des composés selon l'une ou plusieurs des revendications 1 à 4, pour la production d'un médicament destiné au traitement de la résistance à l'insuline.
